(19) Europäisches Patentamt
European Patent Office
Office européen des brevets

(11) Veröffentlichungsnummer: **0 024 562**
**B1**

(12) **EUROPÄISCHE PATENTSCHRIFT**

(45) Veröffentlichungstag der Patentschrift: **25.01.84**

(51) Int. Cl.³: **C 07 C 149/44,** C 07 C 148/00

(21) Anmeldenummer: **80104483.5**

(22) Anmeldetag: **29.07.80**

(54) **Verfahren zur Herstellung der 2,3-Dimercaptopropan-1-sulfonsäure und ihrer Salze.**

(30) Priorität: **16.08.79 DE 2933027**

(43) Veröffentlichungstag der Anmeldung: **11.03.81 Patentblatt 81/10**

(45) Bekanntmachung des Hinweises auf die Patenterteilung: **25.01.84 Patentblatt 84/4**

(84) Benannte Vertragsstaaten: **AT BE CH DE FR GB IT LI LU NL SE**

(56) Entgegenhaltungen:
**CHEMICAL ABSTRACTS, Columbus, Ohio, USA, 1957, Bd. 51 V.E.PETRUN'KIN "Synthesis and properties of dimercapto derivatives of alkanesulfonic acids. I.Synthesis of sodium 2,3-dimercaptopropanesulfonate (Unithiol) and sodium mercaptoethane sulfonate", Abstracts No. 5692l, 5693a**
**JOURNAL OF THE CHEMICAL SOCIETY, London, 1955 N.S. JOHARY + L.N.OWEN "Some Water soluble Derivatives Containing the Sulfonic Acid Group", Seiten 1307-1311**

**Die Akte enthält technische Angaben, die nach dem Eingang der Anmeldung eingereicht wurden und die nicht in dieser Patentschrift enthalten sind.**

(73) Patentinhaber: **Heyl Chemisch-pharmazeutische Fabrik GmbH & Co. KG, Goerzallee 253, D-1000 Berlin 37 (DE)**

(72) Erfinder: **Parr, Wolfgang Dr. Dipl.-Chem., Luzerner Strasse 14, D-1000 Berlin 45 (DE)**

(74) Vertreter: **Kinzebach, Werner, Dr. Patentanwälte et al, Reitstötter J. Prof.Dr.Dr. Kinzebach W. Dr. & Partner Bauerstrasse 22 Postfach 780, D-8000 München 43 (DE)**

**EP 0 024 562 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von 2,3-Dimercaptopropan-1-sulfonsäure und ihrer pharmazeutisch verträglichen Salze

Die Erfindung betrifft ein Verfahren zur Herstellung der 2,3-Dimercaptopropan-1-sulfonsäure der Formel I:

$$\underset{\displaystyle SH}{CH_2}-\underset{\displaystyle SH}{CH}-CH_2-SO_3H \qquad (I)$$

und ihrer pharmazeutisch verträglichen Salze.

Die 2,3-Dimercaptopropan-1-sulfonsäure und deren Salze, insbesondere deren Natriumsalz (nachfolgend als Na-DMPS abgekürzt), sind wertvoll zur Behandlung von Vergiftungen durch hoch toxische Metallsalze. Zu diesen Metallsalzen gehören beispielsweise Quecksilber-, Cadmium- und Bleisalze; die Verbindungen erweisen sich aber auch als wirksam gegen Antimon- und Arsenverbindungen. Die nach dem erfindungsgemässen Verfahren herstellbaren Verbindungen, insbesondere das Na-DMPS weisen einen hohen therapeutischen Index auf und besitzen eine erheblich niedrigere Toxizität als andere Antidota, die bei Vergiftungen durch die genannten Verbindungen eingesetzt werden.

N.S. Johary und L.N. Owen, J.Chem.Soc. (London) 1307 (1955) beschreiben die Herstellung von Na-DMPS durch Umsetzung von intermediär gebildeter 2,3-Dibrompropan-1-sulfonsäure mit Natriumthioacetat. Nachteilig an dieser Arbeitsweise ist jedoch, dass das Natriumthioacetat sehr teuer ist. Ausserdem erhält man nach dem bekannten Verfahren weder reine Produkte, noch befriedigende Ausbeuten.

Gleiches gilt für das in Chemical Abstracts 51, 5692 h-i erwähnte Verfahren von V.E. Petrun'kin. Aus der Originalliteraturstelle ergibt sich, dass das nach diesem Verfahren erhaltene Bleisalzzwischenprodukt relativ stark verunreinigt ist und dass die Gesamtausbeute nur 20% beträgt.

Reine Produkte sind jedoch im Hinblick auf die bestimmungsgemässe Verwendung der 2,3-Dimercaptopropan-1-sulfonsäure und ihrer Salze von wesentlicher Bedeutung. Im Hinblick auf die ausserordentliche Wirksamkeit der genannten Verbindungen und ihre schlechte Zugänglichkeit nach den bekannten Verfahren besteht darüber hinaus ein Bedürfnis nach einem Verfahren zur Herstellung der genannten Verbindungen, das die Erzielung sehr reiner Produkte mit wirtschaftlich tragbaren Ausbeuten erlaubt.

Der Erfindung liegt die Aufgabe zugrunde, ein Verfahren der eingangs genannten Art so auszugestalten, dass es die Erzielung reiner Produkte in hohen Ausbeuten erlaubt. Das Verfahren soll ferner einfach und wirtschaftlich durchführbar sein.

Die Erfindung betrifft daher ein Verfahren zur Herstellung von 2,3-Dimercaptopropan-1-sulfonsäure der Formel I:

$$\underset{\displaystyle SH}{CH_2}-\underset{\displaystyle SH}{CH}-CH_2-SO_3H \qquad (I)$$

und ihrer pharmazeutisch verträglichen Salze durch Umsetzung:

(a) eines Allylhalogenids mit Natriumsulfit;

(b) Bromierung des erhaltenen Prop-2-en-1-sulfonats;

(c) Umsetzung des erhaltenen Natrium-2,3-dibrompropan-1-sulfonats mit Natriumhydrogensulfid in alkalischer Lösung;

(d) Ausfällung des erhaltenen 2,3-Dimercaptopropan-1-sulfonats mit einem $Pb^{2+}$-Salz oder einem anderen Schwermetallsalz,

(e) Freisetzung des Produkts der Formel I mit Schwefelwasserstoff und Isolierung des Produkts, das dadurch gekennzeichnet ist, dass man

- nach der Umsetzung des Allylhalogenids mit Natriumsulfit in Stufe (a) die Reaktionslösung mit Hexan ausschüttelt;
- nach der Bromierung in Stufe (b) überschüssiges Brom mit Natriumsulfit entfernt und mit NaOH einen pH-Wert von 4,5 bis 8 einstellt;
- in Stufe (c) das Natriumhydrogensulfid zehn bis dreissig Stunden bei Raumtemperatur einwirken lässt;
- in Stufe (d) als anderes Schwermetallsalz zur Ausfällung des 2,3-Dimercaptopropan-1-sulfonats ein $Hg^{2+}$, $Cd^{2+}$, $Sn^{2+}$, $Cu^{2+}$, $Ni^{2+}$, $Co^{2+}$ oder $Zn^{2+}$-Salz verwendet und den gebildeten Komplex mit Alkohol ausfällt;
- in Stufe (e) das Fällungsprodukt von Stufe (d) mit Schwefelwasserstoff in Methanol zersetzt und schliesslich die 2,3-Dimercaptopropan-1-sulfonsäure oder ihre Salze isoliert und aus 90%igem Alkohol umkristallisiert, wobei man zwecks Isolierung der Salze nach der Zersetzung in Stufe (e) einen pH-Wert von 4,0 bis 5,5 einstellt.

Besonders vorteilhaft ist es, wenn man im erfindungsgemässen Verfahren:

in Stufe (e) die Zersetzung mit Schwefelwasserstoff in wasserfreiem Medium durchführt;

nach der Zersetzung gemäss Stufe (e) einen pH von 4,5 einstellt;

den pH je nach dem gewünschten Salz mit festem $NaHCO_3$, $NH_4HCO_3$ oder $KHCO_3$, einstellt;

zur Isolierung der 2,3-Dimercaptopropan-1-sulfonsäure oder ihrer Salze das in Stufe (e) erhaltene Filtrat im Vakuum zur Trockne eindampft;

in Stufe (e) aus 90%igem Äthanol oder Isopropanol umkristallisiert, insbesondere

mit einer 10-fachen Gewichtsmenge 90%igem Äthanol oder 90%igem Isopropanol umkristallisiert;

in Stufe (d) bei den Komplexen mit den anderen Schwermetallen nach dem Einstellen des pH auf 4,5 die Ausfällung vornimmt;

die Ausfällung mit 40 bis 70%igem Methanol vornimmt;

die Ausfällung in der Wärme vornimmt und anschliessend mit 50%igem Methanol umfällt;

in Stufe (a) Allylbromid mit Natriumsulfit in Wasser bei etwa 50 bis 100°C umsetzt;

als Metallsalze in Stufe (d) Bleiacetat, Quecksilber-II-Chlorid, Cadmiumchlorid, Zinnchlorid oder Zinksulfat einsetzt, insbesondere eine Lösung von Quecksilber-II-Chlorid in Methanol verwendet.

Zu den Salzen der 2,3-Dimercaptopropan-1-sulfonsäure der obigen Formel I gehören beispielsweise das Natrium-, Kalium-, Ammoniumsalz, die Salze mit primären, sekundären und tertiären Aminen, beispielsweise mit Methylamin, Dimethylamin, Trimethylamin, Äthylamin, Diäthylamin, Polykationische Salze, und Salze mit anderen physiologisch unbedenklichen anorganischen oder organischen Kationen.

Es wird angenommen, dass bei der Umsetzung in Stufe (d) des erfindungsgemässen Verfahrens die in Lösung befindliche Na-DMPS mit Blei-II-Salz ein Doppelsalz der nachfolgenden Formel II bildet:

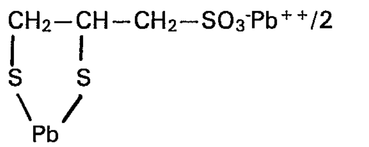

(II)

Es wird angenommen, dass bei der Stufe (e) des erfindungsgemässen Verfahrens ein Komplex der nachfolgenden Formel III gebildet wird:

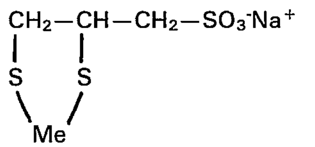

(III)

worin Me für $Hg^{2+}$, $Cd^{2+}$, $Sn^{2+}$, $Cu^{2+}$, $Ni^{2+}$, $Co^{2+}$ oder $Zn^{2+}$ steht.

Das erfindungsgemässe Verfahren vermeidet die Nachteile des bekannten Verfahrens. Man erhält Produkte mit einer Reinheit, die den Anforderungen für die bestimmungsgemässe pharmazeutische Verwendung entspricht und in einer sehr guten Ausbeute.

Erfindungsgemäss kann man im Eintopf-Verfahren arbeiten. So wird das überschüssige Brom in der Stufe (b) des erfindungsgemässen Verfahrens durch Reduktion mit Natriumsulfit anstelle einer Destillation entfernt. Das hat zur Folge, dass die Umsetzung unter Bildung der 2,3-Dibrompropan-1-sulfonsäure sehr bald beendet ist, wobei erhebliche Energie eingespart wird. Die Neutralisation erfolgt mit Natronlauge anstelle von Natriumcarbonat, um Schaumbildung zu vermeiden.

Beim erfindungsgemässen Verfahren hat sich aufgrund kinetischer Untersuchungen gezeigt, dass der SH-Austausch nach 24 ± 5 Stunden bei Raumtemperatur mit einer maximalen Ausbeute an 2,3-Dimercaptopropan-1-sulfonsäure beendet ist. Erhöhte Temperaturen und längere Reaktionszeiten führen immer zur Bildung von unerwünschten Nebenprodukten, hauptsächlich zu gemischten Disulfiden und Tetrasulfiden, die die Endausbeute schmälern und aufgrund ihrer chemischen und physikalischen Eigenschaften nicht ohne grösseren Aufwand aus dem Endprodukt entfernt werden können. Diese Verfahrensweise hat gegenüber dem Stand der Technik den Vorteil, dass das als Zwischenprodukt anfallende Bleisalz schnell zur Verfügung steht und nach analytischen Untersuchungen fast frei von Verunreinigungen ist.

Zur Weiterverarbeitung wird das Bleisalz in der 10-fachen Menge Alkohol, bevorzugt insbesondere Methanol, suspendiert und mit Schwefelwasserstoff zersetzt. Das ausgefallene Bleisulfid wird abfiltriert und zur Ausbeutesteigerung mit Methanol ausgerührt. Die vereinigten Filtrate werden mit festem Natriumhydrogencarbonat auf pH 4,5 eingestellt. Zur Gewinnung des Rohproduktes wird das Filtrat unter Vakuum zur Trockne eingedampft. Man erhält Rohprodukte mit einem DMPS-Gehalt von grösser als 82%. Wesentlich ist bei dieser Variante des erfindungsgemässen Verfahrens, dass das Bleisalz in einem alkoholischen Medium zersetzt wird. Hierdurch erhält man im Vergleich zum Arbeiten in wässrigem Medium sogar eine sprunghafte Steigerung von Ausbeute und Reinheit.

Besonders vorteilhaft ist beim erfindungsgemässen Verfahren, dass man durch Umkristallisation des Rohprodukts aus einem Alkohol-Wassergemisch, bevorzugt 90%igem Äthanol oder 90%igem Isopropanol, ein äusserst reines Produkt erhält. Es ist hervorzuheben, dass es in einfacher Weise gelingt, ein pharmazeutisch reines Endprodukt zu erhalten, wenn man das anfallende Rohprodukt aus der 10-fachen Gewichtsmenge 90%igem Äthanol umkristallisiert.

Nach einer Variante des erfindungsgemässen Verfahrens erfolgt die Umsetzung von intermediär gebildeter 2,3-Dimercaptopropan-1-sulfonsäure mit Metall-II-Salzen der Schwefelwasserstoffgruppe, insbesondere mit Quecksilber-II-Chlorid, Cadmium-II-Chlorid, Kupfer-II-Chlorid und Zinn-II-Chlorid und Metall-II-Salzen der Ammoniumsulfidgruppe, insbesondere mit Zink-II-Sulfat, Nickel-II-Chlorid und Kobalt-II-Chlorid, wobei die entsprechenden Komplexe der Formel III anschliessend isoliert werden.

Die erfindungsgemäss hergestellten Metall-II-DMPS-Verbindungen sind sämtlich 1:1 Komplexe und im Gegensatz zum Bleisalz glatt in Wasser löslich. Sie werden daher aus der wässrigen Lösung nach Einstellen des pH-Wertes auf 4,5 mit einem Alkohol, vorzugsweise Methanol (Alkoholgehalt von 40 bis 70%, vorzugsweise 50%), in der Wärme gefällt, abgesaugt und aus 50%igem Methanol umgefällt, um eventuell mitgefällte anorganische Salze zu entfernen. Die Umfällung ist aber nicht unbedingt erforderlich, da die anorganischen Salze bei der Zersetzung mit Schwefelwasserstoff ungelöst zurückbleiben. Als besonderer Vorteil dieser Arbeitsweise ist hervorzuheben, dass es in einfacher Weise gelingt, stabile Metallkomplexe mit DMPS zu isolieren, die bei der Herstellung und weiteren Verarbeitung auch toxikologisch und ökologisch unbedenkliche Metalle enthalten. Zur weiteren Verarbeitung werden die erfindungsgemäss hergestellten Metall-II-DMPS-Komplexe wie für das Bleisalz beschrieben aufgearbeitet. Man erhält in allen Fällen DMPS und/oder deren Alkalisalze in kristalliner Form und von pharmazeutisch reiner Qualität.

Die nachfolgenden Beispiele dienen zur weiteren Erläuterung der Erfindung.

*Beispiel 1*

Herstellung des Natriumsalzes der 2,3-Dimercaptopropan-1-sulfonsäure

a) Pb-2,3-Pb-Dimercaptopropan-1-sulfonat

121 g (1,0 Mol) frisch destilliertes Allylbromid werden mit 139 g (1,1 Mol) Natriumsulfit in 1 l Wasser unter Rückfluss 4 Stunden lang gekocht, bis die Mischung einphasig ist. Die kalte Lösung wird zweimal mit Hexan extrahiert, um nicht umgesetztes Allylbromid zu entfernen. Die organische Phase wird verworfen. Dann werden innerhalb von 1 1/2 bis 2 Stunden etwa 200 g Brom unter starkem Rühren bei Raumtemperatur bis zur Gelbfärbung hinzugefügt. In die schwach gelbe Lösung wird zur Reduktion des überschüssigen Broms eine Spatelspitze Natriumsulfit gegeben. Die farblose Lösung (pH 0,5) wird mit 25%iger Natriumhydroxidlösung auf pH 6,5 eingestellt. Danach werden 280 g einer 29%igen Natriumhydrogensulfidlösung, die durch Sättigen einer 25%igen Natriumhydroxidlösung mit Schwefelwasserstoff hergestellt wird, langsam unter Rühren zu der noch warmen Lösung hinzugefügt. Nach einer Reaktionszeit unter Rühren von 16 Stunden bei Raumtemperatur wird mit konzentrierter Essigsäure ein pH-Wert von 5 eingestellt und überschüssiger Schwefelwasserstoff im Vakuum abgezogen.

Man entnimmt eine Probe und bestimmt darin jodometrisch den SH-Gehalt (0,67 Mol bezogen auf DMPS). Dann werden 379 g (1 Mol) Bleiacetat × 2 $H_2O$ in 1,5 l Wasser gelöst und auf 60°C erwärmt. Die Bleiacetatlösung wird zu der Reaktionslösung, die ebenfalls auf 60°C erwärmt wird, unter starkem Rühren zugegeben. Man rührt eine Stunde bei 60°C nach, filtriert das Bleisalz ab, wäscht mit 60°C warmem Wasser nach und saugt scharf ab. Zur Reinigung wird das feuchte Bleisalz nochmals in 1 l 60°C warmem Wasser suspendiert, gerührt und wieder filtriert, mit Wasser und Methanol gewaschen und getrocknet.

Ausbeute: 222 g, 66,8% der Theorie bezogen auf titrimetrisch ermitteltes Dithiol.

Gehalt: gef.: 62% Pb, ber.: 62,6% Pb.

b) Na-DMPS

222 g Bleisalz werden in 2 l Methanol suspendiert, und unter starkem Rühren wird Schwefelwasserstoff bis zur Sättigung eingeleitet. Das ausgefallene Bleisulfid wird abfiltriert. Zur Ausbeuteerhöhung wird das Bleisulfid mit 500 ml Methanol ausgerührt und abfiltriert. Die vereinigten Filtrate werden mit Natriumhydrogencarbonat auf pH 4,5 eingestellt, und dann wird nochmals Schwefelwasserstoff eingeleitet. Die Lösung wird nach Kontrolle des pH-Wertes (pH 4,5) filtriert und das klare Filtrat im Vakuum zur Trockne eingedampft.

Ausbeute: 86,5 g Rohprodukt (91,8% d.Th. bezogen auf Bleisalz).

Gehalt: 88,3% SH jodometrisch.

Das Rohprodukt wird aus 865 ml 90%igem Äthanol umkristallisiert und im Vakuum bis zur Gewichtskonstanz getrocknet.

Ausbeute: 65,0 g (69,0% d.Th. bezogen auf Bleisalz, 46,2% d.Th. bezogen auf titrimetrisch ermitteltes Dithiol).

Elementaranalyse:

| | C | H | S | Na |
|---|---|---|---|---|
| gef.: | 17,29 | 3,33 | 45,5 | 10,57 |
| ber.: | 17,13 | 3,35 | 45,75 | 10,93 |

Gehalt: 99,0% SH jodometrisch.

*Beispiel 2*

Herstellung des Ammoniumsalzes der 2,3-Dimercaptopropan-1-sulfonsäure

30 g Bleisalz werden wie in Beispiel 1 b) beschrieben zersetzt und das klare Filtrat mit Ammoniumhydrogencarbonat auf pH 4,5 eingestellt. Es wird nochmals filtriert und im Vakuum zur Trockne eingeengt.

Ausbeute: 8,1 g Rohprodukt (65,3% d.Th. bezogen auf Bleisalz).

Gehalt: 84,1% SH jodometrisch.

Nach Umkristallisieren des Rohproduktes aus 80 ml 90%igem Isopropanol erhält man 5,5 g (44,4% d.Th. bezogen auf Bleisalz).

Gehalt: 95,4% SH jodometrisch.

Elementaranalyse für $C_3H_{11}S_3O_3N$:

| | C | H | S | N |
|---|---|---|---|---|
| gef.: | 17,28 | 5,42 | 45,93 | 6,55% |
| ber.: | 17,55 | 5,40 | 46,85 | 6,82% |

*Beispiel 3*

Herstellung des Kaliumsalzes der 2,3-Dimercaptopropan-1-sulfonsäure

Unter Anwendung der im Beispiel 1 b) und 2 angegebenen Methode und der dort verwendeten Mengen erhält man nach Einstellung des pH-Wertes auf 4,5 mit Kaliumhydrogencarbonat 10 g Rohprodukt (73,2% d.Th. bezogen auf Bleisalz).

Gehalt: 88,3% SH jodometrisch.

Nach Umkristallisieren des Rohproduktes aus 100 ml 87,5%igem Isopropanol erhält man 8 g K-DMPS (58,7% d.Th. bezogen auf Bleisalz; Gehalt: 96,5%).

Elementaranalyse für $C_3H_7S_3O_3K$:

| | C | H | S | K |
|---|---|---|---|---|
| ber.: | 15,92 | 3,12 | 42,49 | 17,27% |
| gef.: | 16,09 | 3,11 | 40,9 | 17,10% |

*Beispiel 4*

Herstellung von Na-DMPS über den Quecksilberkomplex

a) Na-2,3-Hg-Dimercaptopropan-1-sulfonat

Es wird — wie in Beispiel 1 a) beschrieben — bis zur SH-Anlagerung gearbeitet. Jodometrisch wird ein Gehalt von 0,55 Mol DMPS in 1,4 l Lösung ermittelt. Die Lösung wird langsam mit 150 g Quecksilber-II--Chlorid, gelöst in 400 ml Methanol, versetzt. Der pH der Lösung beträgt dann etwa 1. Mit festem Natriumhydrogencarbonat wird auf pH 4,5 eingestellt. Die klare Lösung wird auf 60°C erwärmt und unter starkem Rühren mit 1 l Methanol versetzt, abgekühlt und der Quecksilberkomplex abfiltriert und getrocknet. Rohausbeute 207 g. Zur Reinigung werden 187 g Rohprodukt in 1,7 l Wasser gegeben, mit 1,7 l Methanol versetzt und unter Rühren und Erwärmen in Lösung gebracht. Nach Abkühlen wird der Quecksilberkomplex abfiltriert und getrocknet.

Ausbeute: 157 g (70% bezogen auf titrimetrisch ermitteltes Dithiol).

Nach zweimaligem Umfällen einer kleinen Probe aus Wasser/Methanol ergab die Elementaranalyse für $C_3H_5S_3O_3HgNa$:

| | C | H | S | Hg | Na |
|---|---|---|---|---|---|
| ber.: | 8,81 | 1,23 | 23,53 | 49,06 | 5,62% |
| gef.: | 8,85 | 1,38 | 21,52 | 50,0 | 5,85% |

b) Na-DMPS

Der Quecksilberkomplex wird in 1,5 l Methanol suspendiert, und unter starkem Rühren wird Schwefelwasserstoff bis zur Sättigung eingeleitet. Ausgefallenes Quecksilbersulfid wird abfiltriert. Zur Ausbeuteerhöhung wird das Quecksilbersulfid noch einmal in 500 ml Methanol suspendiert, gerührt und abfiltriert. Die vereinigten Filtrate werden mit festem Natriumhydrogencarbonat auf pH 4,5 eingestellt, nochmals filtriert und im Vakuum zur Trockne eingeengt.

Ausbeute: 67 g Rohprodukt 82,9% d.Th. bezogen auf Quecksilberkomplex.

Gehalt: 88,5% SH jodometrisch.

Nach Umkristallisieren aus 670 ml 90%igem Äthanol erhält man 55,8 g DMPS (69,0% d.Th. bezogen auf Quecksilberkomplex, 48,3% bezogen auf titrimetrisch ermitteltes Dithiol).

Gehalt: 99,6% SH jodometrisch.

*Beispiel 5*

Herstellung von Na-DMPS über den Cadmiumkomplex

a) Na-2,3-Cd-Dimercaptopropan-1-sulfonat

In eine Lösung, die 0,38 Mol DMPS in 2 l Lösung (vgl. Beispiel 1, entspricht Lösung nach Absatz 1) enthält, werden unter Rühren 77 g $CdCl_2 \cdot 1H_2O$ gelöst in 100 ml Wasser zugetropft, und mit festem Natriumhydrogencarbonat wird auf pH 4,5 eingestellt. Die Lösung wird auf 60°C erwärmt und unter starkem Rühren mit 2,1 l Methanol versetzt. Nach dem Abkühlen wird der Cadmiumkomplex abfiltriert, mit Methanol/Wasser (50:50) gewaschen und getrocknet. Rohausbeute: 124 g (das Rohprodukt enthält noch anorganische Salze). Nach Umfällen einer Probe aus Wasser/Methanol ergab die Analyse einen Cadmiumgehalt von 33,8% Cd (35,05% Cd theoretisch).

b) Na-DMPS

Unter Anwendung der in Beispiel 4 b) angegebenen Methode und den dort verwendeten Mengen erhält man, ausgehend von 124 g des rohen Na-2,3--Cd-Dimercaptopropan-1-sulfonat, 65 g Rohprodukt DMPS, 81,4% d.L. bezogen auf titrimetrisch ermitteltes Dithiol.

Gehalt: 81% SH jodometrisch.

Nach dem Umkristallisieren aus 650 ml 90%igem Äthanol erhält man 39,5 g DMPS (49,5% d.Th. bezogen auf titrimetrisch ermitteltes Dithiol).

Gehalt: 98,3% SH jodometrisch.

*Beispiel 6*

Herstellung der Na-DMPS über den Zinnkomplex

a) Na-2,3-Sn-Dimercaptopropan-1-sulfonat

Es wird — wie in Beispiel 1 a) beschrieben — bis zur SH-Anlagerung gearbeitet. Jodometrisch wird ein Gehalt von 0,42 Mol Dithiol in 2 l Lösung ermittelt. Nach dem Verfahren des Beispiels 5 a), jedoch unter Einsatz von 81,6 g Zinn-II-Chlorid, wird der entsprechende Zinnkomplex hergestellt.

Rohausbeute: 100 g (das Rohprodukt enthält noch anorganische Salze).

Die Analyse einer mehrfach umgefällten Probe ergab: 37,52% Sn (36,3% theoretisch).

b) Na-DMPS

Der Zinnkomplex wird, wie in den Beispielen 1 b), 2, 3, 4 b), 5 b) beschrieben, mit Schwefelwasserstoff zersetzt und anschliessend zu Na-DMPS aufgearbeitet.

Ausbeute: 58,5 g Rohprodukt (66,3% d.Th. bezogen auf titrimetrisch ermitteltes Dithiol).

Gehalt: 83% SH jodometrisch.

Nach Umkristallisieren aus 585 ml 90%igem Äthanol erhält man 42,1 g DMPS (47,7% d.Th. bezogen auf titrimetrisch ermitteltes Dithiol).

Gehalt: 98,2% SH jodometrisch.

*Beispiel 7*

Herstellung von Na-DMPS über den Zinkkomplex

a) Na-2,3-Zn-Dimercaptopropan-1-sulfonat

Es wird — wie in Beispiel 1 a) beschrieben — bis zur SH-Anlagerung gearbeitet. Der jodometrisch ermittelte Gehalt beträgt 0,32 Mol Dithiol in 2 l Lösung. Nach der in Beispiel 5 a) beschriebenen Methode wird unter Einsatz von 92 g Zink-II-Sulfat der Zinkkomplex hergestellt.

Ausbeute: 104 g Rohprodukt.

Nach Umfällen aus Wasser/Methanol ergab die Metallanalyse: 23,47% Zn (gef.); 23,89% Zn (theor.).

b) Na-DMPS

100 g Zinkkomplex werden in 1 l Methanol suspendiert, mit konz. Ammoniak deutlich alkalisch gemacht und durch Einleiten von Schwefelwasserstoff zersetzt. Das ausgefallene Zinksulfid wird abfiltriert, mit 500 ml Methanol ausgerührt und die Filtrate vereinigt. Nach Ansäuern mit Essigsäure auf pH 4,5 wird zur Trockne eingeengt.

Rohausbeute: 43 g (63,9% d.Th. bezogen auf titrimetrisch ermitteltes Dithiol).

Gehalt: 83,2% SH jodometrisch.

Das Rohprodukt wird aus 430 ml 90%igem Äthanol umkristallisiert.

Ausbeute: 31,3 g (46,6% d.Th. bezogen auf titrimetrisch ermitteltes Dithiol).

Gehalt: 97,5% SH jodometrisch.

**Patentansprüche**

1. Verfahren zur Herstellung von 2,3-Dimercaptopropan-1-sulfonsäure der Formel I:

$$\underset{|\atop SH}{CH_2}-\underset{|\atop SH}{CH}-CH_2-SO_3H \qquad (I)$$

und ihrer pharmazeutisch verträglichen Salze durch Umsetzung:

(a) eines Allylhalogenids mit Natriumsulfit;

(b) Bromierung des erhaltenen Prop-2-en-1-sulfonats;
(c) Umsetzung des erhaltenen Natrium-2,3-dibrompropan-1-sulfonats mit Natriumhydrogensulfid in alkalischer Lösung;
(d) Ausfällung des erhaltenen 2,3-Dimercaptopropan-1-sulfonats mit einem $Pb^{2+}$-Salz oder einem anderen Schwermetallsalz,
(e) Freisetzung des Produkts der Formel I mit Schwefelwasserstoff und Isolierung des Produkts, dadurch gekennzeichnet, dass man:
- nach der Umsetzung des Allylhalogenids mit Natriumsulfit in Stufe (a) die Reaktionslösung mit Hexan ausschüttelt;
- nach der Bromierung in Stufe (b) überschüssiges Brom mit Natriumsulfit entfernt und mit NaOH einen pH-Wert von 4,5 bis 8 einstellt;
- in Stufe (c) das Natriumhydrogensulfid zehn bis dreissig Stunden bei Raumtemperatur einwirken lässt;
- in Stufe (d) als anderes Schwermetallsalz zur Ausfällung des 2,3-Dimercaptopropan-1-sulfonats ein $Hg^{2+}$, $Cd^{2+}$, $Sn^{2+}$, $Cu^{2+}$, $Ni^{2+}$, $Co^{2+}$ oder $Zn^{2+}$-Salz verwendet und den gebildeten Komplex mit Alkohol ausfällt;
- in Stufe (e) das Fällungsprodukt von Stufe (d) mit Schwefelwasserstoff in Methanol zersetzt und schliesslich die 2,3-Dimercaptopropan-1-sulfonsäure oder ihre Salze isoliert und aus 90%igem Äthanol umkristallisiert, wobei man zwecks Isolierung der Salze nach der Zersetzung in Stufe (e) einen pH-Wert von 4,0 bis 5,5 einstellt.

2. Verfahren nach Anspruch 1, dadurch gekennzeichnet, dass man nach der Zersetzung in Stufe (e) einen pH-Wert von 4,5 einstellt, vorzugsweise mit festem $NaHCO_3$, $NH_4HCO_3$ oder $KHCO_3$, je nachdem welches Salz erwünscht ist.

3. Verfahren nach Anspruch 1, dadurch gekennzeichnet, dass man zur Isolierung der 2,3-Dimercaptopropan-1-sulfonsäure oder ihrer Salze das in Stufe (e) erhaltene Filtrat im Vakuum zur Trockne eindampft und den Rückstand aus einer 10-fachen Gewichtsmenge 90%igem Äthanol, oder 90%igem Isopropanol umkristallisiert.

4. Verfahren nach Anspruch 1, dadurch gekennzeichnet, dass man in Stufe (d) bei den Komplexen mit den anderen Schwermetallen nach dem Einstellen des pH auf 4,5 die Ausfällung vornimmt.

5. Verfahren nach Anspruch 4, dadurch gekennzeichnet, dass man mit 40 bis 70%igem Methanol ausfällt.

6. Verfahren nach einem der Ansprüche 4 oder 5, dadurch gekennzeichnet, dass man in der Wärme ausfällt und anschliessend mit 50%igem Methanol umfällt.

7. Verfahren nach Anspruch 1, dadurch gekennzeichnet, dass man in Stufe (a) Allylbromid mit Natriumsulfit in Wasser bei 50 bis 100°C umsetzt.

8. Verfahren nach Anspruch 1, dadurch gekennzeichnet, dass man als Metallsalz in Stufe (d) Bleiacetat, Quecksilber-II-Chlorid, Cadmiumchlorid, Zinnchlorid oder Zinksulfat einsetzt, insbesondere eine Lösung von Quecksilber-II-Chlorid in Methanol verwendet.

**Claims**

1. A process for preparing 2,3-dimercaptopropane-1-sulfonic acid having the formula:

$$\begin{array}{llll} CH_2- & CH- & CH_2- & SO_3H \\ | & | & & \\ SH & SH & & \end{array} \quad (I)$$

and the pharmaceutically acceptable salts thereof, by
(a) reacting an allyl halide with sodium sulfite;
(b) brominating the obtained 2-propene-1-sulfonate;
(c) reacting the obtained sodium-2,3-dibromopropane-1-sulfonate with sodium hydrogen sulfide in alkaline solution;
(d) precipitating the obtained sodium-2,3-mercaptopropane-1-sulfonate with a $Pb^{2+}$ salt or another heavy metal salt;
(e) liberating the product having the formula I with hydrogen sulfide and isolating the product, characterized in that
- the reaction solution obtained after the reaction of the allyl halide with sodium sulfite in step (a) is extracted with hexane;
- excessive bromine after the bromination in step (b) is removed with sodium sulfite and the pH is adjusted with NaOH to 4.5 to 8;
- the sodium hydrogen sulfide in step (c) is allowed to react 10 to 30 hours at room temperature;
- a $Hg^{2+}$, $Cd^{2+}$, $Sn^{2+}$, $Cu^{2+}$, $Ni^{2+}$, $Co^{2+}$ or $Zn^{2+}$ salt is used as another heavy metal salt for the precipitation of 2,3-dimercaptopropane-1-sulfonate in step (d) and the formed complex is precipitated with alcohol;
- in step (e) the precipitate of step (d) is decomposed with hydrogen sulfide in methanol and finally the 2,3-dimercaptopropane-1-sulfonic acid or its salts is isolated and recrystallized from 90% ethanol, thereby the pH is adjusted to 4.0 to 5.5 for the isolation of the salts after the decomposition in step (e).

2. Process according to claim 1, characterized in that after the decomposition in step (e) the pH is adjusted to 4.5, preferably using solid $NaHCO_3$, $NH_4HCO_3$ or $KHCO_3$, dependent on which salt is desired.

3. Process according to claim 1, characterized in that for the isolation of 2,3-dimercaptopropane-1-sulfonic acid or its salts the filtrate obtained from step (e) is concentrated to dryness in vacuo and the residue is recrystallized from the 10-fold quantity by weight of 90% ethanol or 90% isopropanole.

4. Process according to claim 1, characterized in that in step (d) the precipitation of the complexes formed with other heavy metals is carried out after the pH has been adjusted to 4.5.

5. Process according to claim 4, characterized in that the precipitation is effected with 40 to 70% methanol.

6. Process according to one of claims 4 or 5, characterized in that the precipitation is effected from warm solution and the precipitate is subsequently recrystallized from 50% methanol.

7. Process according to claim 1, characterized in that in step (a) allyl bromide is reacted with sodium sulfite in water at a temperature of 50 to 100°C.

8. Process according to claim 1, characterized in that lead acetate, mercury-II-chloride, cadmium chloride, tin chloride or zinc sulfate, especially a solution of mercury-II-chloride in methanol, is used as metal salt in step (d).

**Revendications**

1. Procédé de préparation de l'acide 2,3-dimercaptopropane-1-sulfonique de formule I:

$$\underset{SH}{\underset{|}{CH_2}}-\underset{SH}{\underset{|}{CH}}-CH_2-SO_3H \qquad (I)$$

et de ses sels pharmaceutiquement acceptables par réaction:

(a) d'un halogénure d'allyle avec du sulfite de sodium;
(b) bromuration du prop-2-ène-1-sulfonate obtenu;
(c) réaction du 2,3-dibromopropane-1-sulfonate de sodium avec l'hydrogénosulfure de sodium en solution alcaline;
(d) précipitation du 2,3-dimercaptopropane-1-sulfonate avec un sel de $Pb^{2+}$ ou un autre sel de métal lourd;
(e) libération du produit de formule I avec l'acide sulfhydrique et isolement du produit, caractérisé par le fait:

- qu'après la réaction de l'halogénure d'allyle avec le sulfite de sodium dans l'étape (a) on extrait la solution réactionnelle avec de l'hexane;
- qu'après la bromuration dans l'étape (b) on élimine l'excès de brome avec du sulfite de sodium et on établit un pH de 4,5 à 8 à l'aide de NaOH;
- qu'à l'étape (c) on laisse agir l'hydrogénosulfure de sodium pendant 10 à 30 heures à température ambiante;
- qu'à l'étape (d) on utilise comme autre sel de métal lourd pour précipiter le 2,3-dimercaptopropane-1-sulfonate un sel de $Hg^{2+}$, $Cd^{2+}$, $Sn^{2+}$, $Cu^{2+}$, $Ni^{2+}$, $Co^{2+}$ ou $Zn^{2+}$ et que l'on précipite le complexe obtenu par l'alcool;
- qu'à l'étape (e) on décompose le produit précipité de l'étape (d) avec de l'acide sulfhydrique dans le méthanol et que, finalement, on isole et recristallise dans l'éthanol à 90% l'acide 2,3-dimercaptopropane-1-sulfonique ou ses sels étant entendu que dans le but d'isoler les sels après la décomposition dans l'étape (e) on établit le pH à une valeur de 4,0 à 5,5.

2. Procédé selon la revendication 1, caractérisé par le fait qu'après la décomposition dans l'étape (e) on établit le pH à une valeur de 4,5, de préférence avec du bicarbonate de sodium, du bicarbonate d'ammonium ou du bicarbonate de potassium solide, selon le sel que l'on désire.

3. Procédé selon la revendication 1, caractérisé par le fait que pour isoler l'acide 2,3-dimercaptopropane-1-sulfonique ou ses sels, on évapore à sec sous vide le filtrat obtenu dans l'étape (e) et on recristallise le résidu dans dix fois son poids d'éthanol à 90% ou d'isopropanol à 90%.

4. Procédé selon la revendication 1, caractérisé par le fait que dans l'étape (d) on effectue la précipitation des complexes des autres métaux lourds après établissement du pH à 4,5.

5. Procédé selon la revendication 4, caractérisé par le fait que l'on effectue la précipitation avec du méthanol à 40 à 70%.

6. Procédé selon la revendication 4 ou 5, caractérisé par le fait que l'on effectue la précipitation à chaud et finalement reprécipite avec du méthanol à 50%.

7. Procédé selon la revendication 1, caractérisé par le fait que dans l'étape (a) on fait réagir le bromure d'allyle avec le sulfite de sodium dans l'eau à une température de 50 à 100°C.

8. Procédé selon la revendication 1, caractérisé par le fait que l'on introduit comme sel métallique dans l'étape (d) de l'acétate de plomb, du chlorure de mercure-II, du chlorure de cadmium, du chlorure d'étain ou du sulfate de zinc, et qu'en particulier on utilise une solution de chlorure de mercure-II dans le méthanol.